# EUROPEAN PATENT APPLICATION

(11) **EP 4 197 477 A1**
(43) Date of publication of application: **21.06.2023**
(21) Application number: 22163216.9
(22) Date of filing: 21.03.2022
(51) Int. Cl.: A61B 34/20, A61B 17/00, A61B 90/00

(54) **VERIFICATION BLOCK STRUCTURE AND VERIFICATION SYSTEM FOR ORTHOPEDIC SURGERY**

(30) Priority: 16.12.2021 TW 110147110
(71) Applicant: Point Robotics (Singapore) Pte. Ltd., Singapore 068898 (SG)
(72) Inventor: HO, Ming-Chun, 30261 Zhubei City, Hsinchu County (TW); HSIEH, Chih-Hsiang, 30261 Zhubei City, Hsinchu County (TW); WU, Chao-Wei, 30261 Zhubei City, Hsinchu County (TW); YEN, Chia-Ho, 30261 Zhubei City, Hsinchu County (TW); WANG, Wen-Teng, 30261 Zhubei City, Hsinchu County (TW); JUANG, Shyue-Cherng, 30261 Zhubei City, Hsinchu County (TW)
(74) Representative: Viering, Jentschura & Partner mbB Patent- und Rechtsanwälte

(57) **Abstract**

A verification block structure (1) and a verification system (2) for orthopedic surgery are provided. The verification block structure (1) includes a base (11) and an artificial bone block (131, 132, 133). The base (11) has a carrying portion (111) and a bottom (113) corresponding to the carrying portion (111). The artificial bone block (131, 132, 133) is detachably fixed to the carrying portion (111) of the base (11), and a shape or a material of the artificial bone block (131, 132, 133) is determined upon a bone characteristic of a patient and/or a surgical method.

## Description

### FIELD OF THE DISCLOSURE

The present disclosure relates to a verification block structure and a verification system, and more particularly to a verification block structure and a verification system for an orthopedic surgery.

### BACKGROUND OF THE DISCLOSURE

The use of surgical robots for surgery has gradually become a trend. To keep operations of the surgical robot at a state of high precision and low error, it is necessary to periodically calibrate the surgical robot, so as to ensure that there is no deviation between the surgical instrument and a surgical site during the surgery. In addition to periodical calibrations, it is also necessary to verify, for an orthopedic surgical robot, that a drilling depth set by the system is the same as a depth actually achieved when drilling bones in complex surgical situations. Therefore, ensuring that the system setting value is consistent with the actual value is a challenging issue in the related art.

### SUMMARY OF THE DISCLOSURE

In response to the above-referenced technical inadequacies, the present disclosure provides a verification block structure for an orthopedic surgery, in which the verification block structure adaptively includes an artificial bone block for preoperative simulation and verification.

In one aspect, the present disclosure provides a verification block structure for an orthopedic surgery, and the verification block structure includes a base and an artificial bone block. The base has a carrying portion and a bottom corresponding to the carrying portion. The artificial bone block is detachably fixed to the carrying portion of the base, and a shape or a material of the artificial bone block is determined upon a bone characteristic of a patient and/or a surgical method.

In another aspect, the present disclosure provides a verification system for an orthopedic surgery, and the verification system includes a surgical instrument, a verification block structure, and a calculation and detection unit. A base of the verification block structure has a carrying portion and a bottom corresponding to the carrying portion. The artificial bone block is detachably fixed to the carrying portion of the base, and a shape or a material of the artificial bone block is determined upon a bone characteristic of a patient and/or a surgical method. The calculation and detection unit is configured to detect a position of the surgical instrument, so as to compute simulation data through using the surgical instrument to perform a surgery on the verification block according to the surgical method.

These and other aspects of the present disclosure will become apparent from the following description of the embodiment taken in conjunction with the following drawings and their captions, although variations and modifications therein may be affected without departing from the spirit and scope of the novel concepts of the disclosure.

### BRIEF DESCRIPTION OF THE DRAWINGS

The described embodiments may be better understood by reference to the following description and the accompanying drawings, in which:
FIGS. 1A to 1C are schematic diagrams of a verification block structure according to one embodiment of the present disclosure; and
FIG. 2 is a schematic diagram of a verification system according to one embodiment of the present disclosure.

### DETAILED DESCRIPTION OF THE EXEMPLARY EMBODIMENTS

The present disclosure is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a", "an", and "the" includes plural reference, and the meaning of "in" includes "in" and "on". Titles or subtitles can be used herein for the convenience of a reader, which shall have no influence on the scope of the present disclosure.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present disclosure or of any exemplified term. Likewise, the present disclosure is not limited to various embodiments given herein. Numbering terms such as "first", "second" or "third" can be used to describe various components, signals or the like, which are for distinguishing one component/signal from another one only, and are not intended to, nor should be construed to impose any substantive limitations on the components, signals or the like.

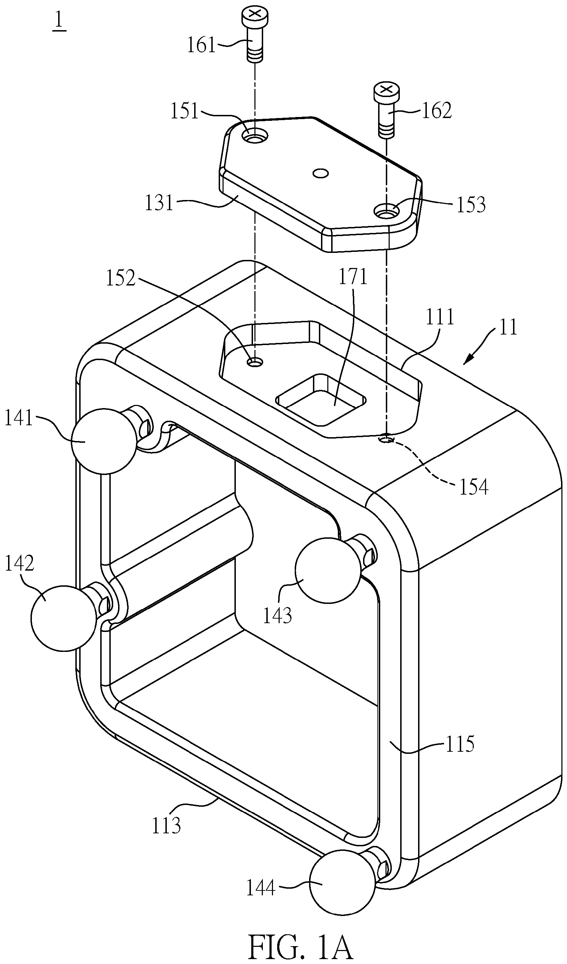

Referring to FIGS. 1A to 1C, which are schematic diagrams of a verification block structure according to one embodiment of the present disclosure. Referring first to FIG. 1A, a verification block structure 1 includes a base 11 and an artificial bone block 131. The base 11 has a carrying portion 111 and a bottom 113 corresponding to the carrying portion 111. A side surface 115 of the base 11 is connected to the carrying portion 111 and the bottom 113. The artificial bone block 131 is detachably fixed on the carrying portion 111 of the base 11, and a shape or a material of the artificial bone block 131 is determined upon a bone characteristic of a patient and/or a surgical method. In this embodiment, the bone characteristic is a bone density, bone hardness or a bone pathological feature, and the surgical method is drilling, cutting, scraping, or grinding.

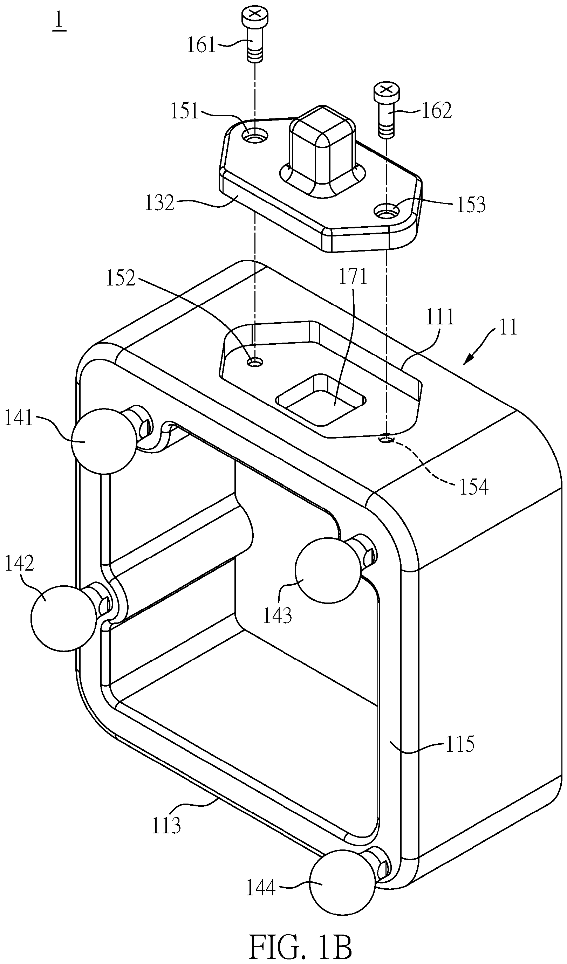

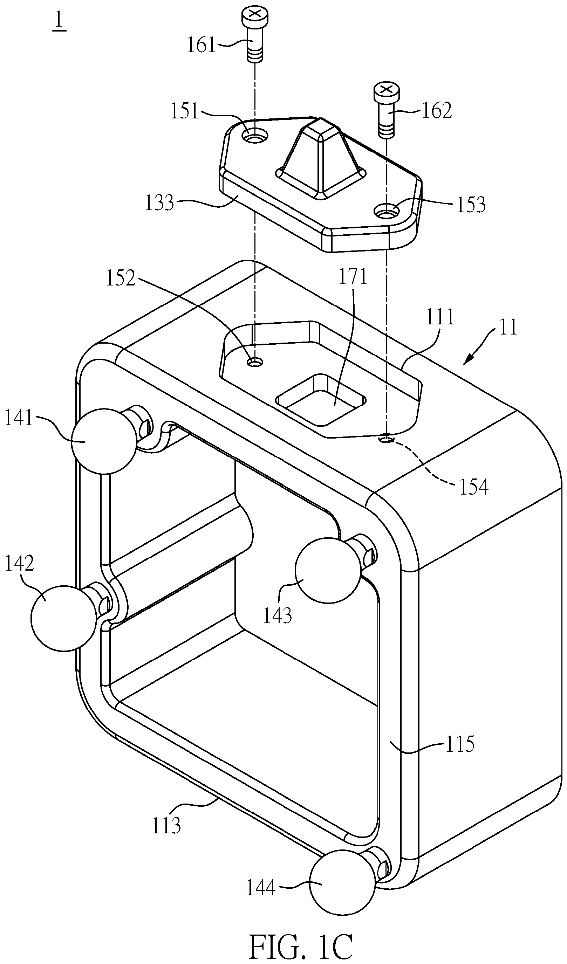

Specifically, a user (e.g., a surgeon) can select the most suitable one from a plurality of artificial bone blocks with different shapes or different materials according to bone characteristics of the patient and/or surgical methods, and then fix the selected artificial bone block on the carrying portion 111 of the base 11. For example, the artificial bone blocks 131, 132 and 133 in FIGS. 1A to 1C have different shapes which would be respectively adapting to drilling, cutting and grinding operations, but the present disclosure is not limited thereto. For the drilling operation, the user can select the artificial bone block 131 with a specific shape, such that the artificial bone block 131 can be fixed on the carrying portion 111 of the base 11, as shown in FIG. 1A. For cutting or grinding operations, the user can select the artificial bone block 132 or 133 with other shape, so as to fix the selected artificial bone block on the carrying portion 111 of the base 11, as shown in FIG. 1B or FIG. 1C.

In other embodiments, it is also feasible to provide multiple artificial bone blocks with different materials for respectively adapting to patients with different bone characteristics, but the present disclosure is not limited thereto. For example, each of the artificial bone blocks 131 to 133 in FIGS. 1A to 1C may be made of varied materials. In the drilling operation, a user can select the most suitable one from the plurality of artificial bone blocks 131 with varied materials according to bone characteristics of the patient, so as to fix the selected artificial bone block on the carrying portion 111 of the base 11. Similarly, in the cutting or grinding operation, the user can also select the most suitable one from the artificial bone blocks 132 or 133 with varied materials according to the bone characteristics of the patient, so as to fix the selected artificial bone block on the carrying portion 111 of the base 11.

In other words, the verification block structure 1 of the present disclosure can be provided with a suitable artificial bone block for preoperative simulation and verification. In addition, the artificial bone block imitates a bone part of the patient, and is used to simulate and verify the drilling, cutting, scraping or grinding operation of the bone part. Since the bone part of the patient has an external structure part and an internal structure part, the artificial bone block similarly includes an outer part and a center part. Hardness of the outer part corresponds to a bone density of the external structure part, and hardness of the center part corresponds to a bone density of the internal structure part, but the present disclosure is not limited thereto. In addition, the artificial bone block can also be used to simulate a tissue structure or a shape of a bone spur, a lamina, or a cartilaginous endplate.

In practical application, before performing a surgery on the patient, a tomography equipment, a magnetic resonance imaging equipment, an ultrasound equipment etc. can be used to scan a specific bone part (such as the vertebrae) of the patient, and obtained scan images can be used to determine a distribution of bone density and a bone shape as to the specific bone part. According to the measured distribution and the measured bone shape, materials with similar densities (such as bone cement, gypsum, and ceramics, or rigid polyurethane foam selected for testing orthopedic devices and instruments conforming to the American Society for Testing and Materials (ASTM) International F1839 specification, such as sawbones) can be used, to create the artificial bone blocks with a similar distribution and a similar bone shape. Or, the user may select a suitable artificial bone block from multiple standard templates in terms of the measured distribution and the measured bone shape. Afterwards, a surgical instrument is utilized to perform the drilling, cutting, scraping or grinding operation on the artificial bone block with. By means of such a verification procedure, before the preplanned surgical method is actually implemented, we can know in advance whether the surgical result is likely to meet the surgical plan or whether the surgical result is the same as simulation, calculation done by the surgical system. That is, for a planned drilling depth, a planned grinding volume, etc. (simulation data), whether actually measured drilling depth, grinding volume, etc. is likely to be the same as those planned or computed by the surgical system during the actual operation. In addition, the surgeon can also simulate the operation process through such a verification procedure, thereby allowing the surgeon to practice how the operation is to be performed in advance.

In practice, a surgeon or a surgical robotic system can use surgical instruments to conduct surgery exercise on the verification block structure 1. However, it is necessary to track position of the verification block structure 1 before using the surgical instruments. Therefore, the verification block structure 1 further includes a plurality of marking elements 141, 142, 143 and 144 which are used for position tracking. The marking elements 141 to 144 are disposed on the side surface 115 of the base 11, as shown in FIGS. 1A to 1C.

In the present embodiment, the marking elements 141 to 144 can be infrared reflective balls, but the present disclosure is not limited thereto. Since position tracking of the marking elements 141 to 144 are known to those skilled in the art, the details thereof shall not be repeated herein. It should be noted that, in order to detachably fix the artificial bone blocks 131 to 133 on the carrying portion 111, the artificial bone blocks 131 to 133 and the carrying portion 111 can be provided with a plurality of fastening points at corresponding positions (for example, screw locking points 151, 152, 153 and 154 of FIGS. 1A to 1C), such that the user can detachably fix the artificial bone blocks 131, 132, 132, and 133 to the carrying portion 111 by using at least one fastener (e.g., screws 161 and 162 in FIGS. 1A to 1C).

In addition, as shown in FIG. 1A to FIG. 1C, a mortise 171 can be further formed on the carrying portion 111, and the artificial bone blocks 131, 132 and 133 each have a tenon portion corresponding to the mortise 171, such that the user can also use the mortise 171 and the tenon portion to detachably fix the artificial bone blocks 131, 132, and 133 on the carrying portion 111, but the present disclosure does not limit specific implementations for detachably fixing the artificial bone blocks 131, 132, and 133 on the carrying portion 111.

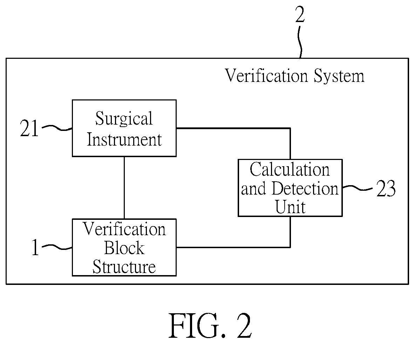

On the other hand, reference is made to FIG. 2, which is a schematic diagram of a verification system according to one embodiment of the present disclosure. As shown in FIG. 2, a verification system 2 includes a surgical instrument 21, a verification block structure 1 and a calculation and detection unit 23, of which the details of the verification block structure 1 will not be repeated hereinafter. Specifically, a surgeon or a surgical robot system can use the surgical instrument 21 of the verification system 2 to perform a surgery exercise on the verification block structure 1 according to the aforementioned surgical method. In addition, the calculation and detection unit 23 can be implemented by hardware (e.g., a processor and a memory) combined with software and/or firmware, but the present disclosure does not limit the specific implementation of the calculation and detection unit 23. The calculation and detection unit 23 is configured to detect a position of the surgical instrument 21, so as to compute simulation data after using the surgical instrument 21 to perform a surgery on the verification block 1 according to the surgical method. In this embodiment, the simulation data is a drilling area, a drilling depth, a cutting area, a cutting volume, a scraping area, a scraping volume, a grinding area or a grinding volume, but the present disclosure is not limited thereto.

For example, when the user selects the artificial bone block 131 of FIG. 1A for simulating and verifying the drilling of a bone part of the patient, the calculation and detection unit 23 is configured to detect a position of the surgical instrument 21 (e.g., a drilling machine), so as to compute a drilling area or a drilling depth after the drilling operation is performed on the artificial bone block 131 by the drilling machine. In addition, when the user selects the artificial bone block 132 of FIG. 1B for simulating and verifying the cutting of a bone part of the patient, the calculation and detection unit 23 can detect a position of the surgical instrument 21 (e.g., a cutting machine), so as to compute a cutting area or a cutting volume after the cutting operation is performed on the artificial bone block 132 by the cutting machine.

Similarly, when the user selects the artificial bone block 133 of FIG. 1C for simulating and verifying the grinding of a bone part of the patient, the calculation and detection unit 23 can detect a position of the surgical instrument 21 (e.g., a grinding machine), so as to compute a grinding area or a grinding volume after the grinding operation is performed on the artificial bone block 133 by the grinding machine. In the present embodiment, the calculation and detection unit 23 can go along with an optical tracker (not shown in FIG. 2), to detect a position of the surgical instrument 21 according to a spatial positioning of the verification block structure 1, but the present disclosure does not limit specific implementations for the calculation and detection unit 23 to detect the position of the surgical instrument 21 and compute the above simulation data.

In conclusion, one advantage of the present disclosure is that in the verification block structure and the verification system provided by the present disclosure, a shape or a material of the artificial bone block can be determined upon a bone characteristic of a patient and/or a surgical method, thereby improving credibility of the preoperative simulation and verification. In addition, the artificial bone block is detachably fixed on the carrying portion of the base, such that the user only needs to replace the artificial bone block to perform surgical simulations according to different surgical methods or conditions without having to do position tracking again, hence increasing convenience for simulating surgical operations.

The foregoing description of the exemplary embodiments of the disclosure has been presented only for the purposes of illustration and description and is not intended to be exhaustive or to limit the disclosure to the precise forms disclosed. Many modifications and variations are possible in light of the above teaching.

The embodiments were chosen and described in order to explain the principles of the disclosure and their practical application so as to enable others skilled in the art to utilize the disclosure and various embodiments and with various modifications as are suited to the particular use contemplated. Alternative embodiments will become apparent to those skilled in the art to which the present disclosure pertains without departing from its spirit and scope.

## Claims

1. A verification block structure (1) for an orthopedic surgery, **characterized by** comprising:
a base (11) having a carrying portion (111) and a bottom (113) corresponding to the carrying portion (111); and
an artificial bone block (131, 132, 133) detachably fixed to the carrying portion (111) of the base (11), wherein a shape or a material of the artificial bone block (131, 132, 133) is determined upon a bone characteristic of a patient and/or a surgical method.

2. The verification block structure (1) according to claim 1, **characterized in that** the base (11) further includes a side surface (115) that is connected to the carrying portion (111) and the bottom (113), and the verification block structure (1) further includes a plurality of marking elements (141, 142, 143, 144) which are disposed on the side surface (115) of the base (11) and used for position tracking.

3. The verification block structure (1) according to claim 1, **characterized in that** the bone characteristic is a bone density, bone hardness or a bone pathological feature, and the surgical method is drilling, cutting, scraping or grinding.

4. The verification block structure (1) according to claim 3, **characterized in that** the artificial bone block (131, 132, 133) imitates a bone part of the patient, and is used to simulate and verify the drilling, cutting, scraping or grinding of the bone part.

5. The verification block structure (1) according to claim 4, **characterized in that** the bone part of the patient has an external structure part and an internal structure part, the artificial bone block (131, 132, 133) includes an outer part and a center part, and hardness of the outer part corresponds to a bone density of the external structure part, and hardness of the center part corresponds to a bone density of the internal structure part.

6. A verification system (2) for an orthopedic surgery, **characterized by** comprising:
a surgical instrument (21);
a verification block structure (1), including:
a base (11) having a carrying portion (111) and a bottom (113) corresponding to the carrying portion (111); and
an artificial bone block (131, 132, 133) detachably fixed to the carrying portion (111) of the base (11), wherein a shape or a material of the artificial bone block (131, 132, 133) is determined upon a bone characteristic of a patient and/or a surgical method; and
a calculation and detection unit (23) configured to detect a position of the surgical instrument (21), so as to compute simulation data through using the surgical instrument (21) to perform a surgery on the verification block according to the surgical method.

7. The verification system (2) according to claim 6, **characterized in that** the base (11) further includes a side surface (115) that is connected to the carrying portion (111) and the bottom (113), and the verification block structure (1) further includes a plurality of marking elements (141, 142, 143, 144) which are disposed on the side surface (115) of the base (11) and used for position tracking.

8. The verification system (2) according to claim 6, **characterized in that** the bone characteristic is a bone density, bone hardness or a bone pathological feature, the surgical method is drilling, cutting, scraping or grinding, and the simulation data is a drilling area, a drilling depth, a cutting area, a cutting volume, a scraping area, a scraping volume, a grinding area or a grinding volume.

9. The verification system (2) according to claim 8, **characterized in that** the artificial bone block (131, 132, 133) imitates a bone part of the patient, and is used to simulate and verify the drilling, cutting, scraping or grinding of the bone part.

10. The verification system (2) according to claim 9, **characterized in that** the bone part of the patient has an external structure part and an internal structure part, the artificial bone block (131, 132, 133) includes an outer part and a center part, and hardness of the outer part corresponds to a bone density of the external structure part, and hardness of the center part corresponds to a bone density of the internal structure part.
